# FASCICULE DE BREVET EUROPEEN

(11) **EP 0 956 010 B1**
(45) Date de publication et mention de la délivrance du brevet: **17.04.2002**
(21) Numéro de dépôt: 97943943.7
(22) Date de dépôt: 03.10.1997
(51) Int. Cl.: A61K 31/19, A61K 9/16, A61P 25/08

(54) **MICROSPHERES PHARMACEUTIQUES D'ACIDE VALPROIQUE POUR ADMINISTRATION ORALE**
VALPROINSÄURE ENTHALTENDE MIKROSPHÄREN FÜR ORALE VERABREICHUNG
PHARMACEUTICAL MICROSPHERES OF VALPROIC ACID FOR ORAL ADMINISTRATION

(30) Priorité: 07.10.1996 FR 9612201
(43) Date de publication de la demande: 17.11.1999
(73) Titulaire: SANOFI-SYNTHELABO, 75635 Paris Cédex 13 (FR)
(72) Inventeur: LANGLOIS, Christian, F-91370 Verrières-le-Buisson (FR); LANNE, Jean-Yves, F-33000 Bordeaux (FR)
(74) Mandataire: Bernasconi, Jean Raymond
(86) Numéro de dépôt international: FR9701762
(87) Numéro de publication internationale: WO9815268

(56) Documents cités:
- FR-A- 2 293 974
- FR-A- 2 657 257
- FR-A- 2 682 677
- GIANNOLA L. I. ET AL: "Preparation of withe beeswax microspheres loaded with valproic acid and kinetic study of drug release" DRUG DEV. AND IND. PHARMACY, vol. 21, no. 7, 1995, pages 793-807, XP000677159 cité dans la demande
- PHARMAZIE, vol. 48, no. 12, 1993, pages 917-920, XP000415619
- GIANNOLA L.I. ET AL: "Carnauba Wax Microspheres loaded with Valproic Acid : Preparation and Evaluation of Drug Release" DRUG DEV. AND IND. PHARMACY, vol. 21, no. 13, 1995, pages 1563-1572, XP000677299

## Description

La présente invention concerne dune manière générale de nouvelles microsphères pharmaceutiques pour administration orale.

En particulier, l'invention se rapporte à de nouvelles microsphères pharmaceutiques pour administration orale contenant l'acide valproïque de formule : 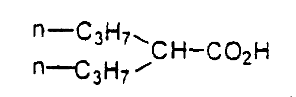 et un de ses sels pharmaceutiquement acceptables, en particulier un sel de métal alcalin tel que le sodium ou le potassium, ou alcalino-terreux. tel que le calcium ou le magnésium.

Le valproate de sodium constitue un médicament antiépileptique largement commercialisé à l'heure actuelle notamment sous forme de comprimés titrant 500 mg par unité d'administration.

Ces comprimés, qui sont munis d'un enrobage entérique ou à libération programmée, se présentent, pour cette raison, sous un volume relativement important.

En conséquence, de tels comprimés feront généralement l'objet d'inconvénients notamment pour les enfants ou les personnes éprouvant des difficultés de déglutition par exemple des personnes âgées.

Pour ce type de patient, il apparaît donc souhaitable de disposer de formes d'administration mieux adaptées à leur état ou à feur condition.

Par ailleurs, le valproate de sodium est doté d'un goût amer assez désagréable. Il s'avèrera, par conséquent, nécessaire de tenir compte de cet inconvénient pour les formes d'administration adaptées à l'enfant par exemple les formes sirop ou les solutés buvables en masquant ce goût par divers artifices.

Toutefois, des formes d'administration telles que des sirops ne permettent pas de faire bénéficier l'enfant des avantages qu'apportent dans certains cas la gastro-résistance et la libération prolongée de l'ingrédient actif.

D'autre part, l'ajustement de la dose d'ingrédient actif au poids de l'enfant en respectant la posologie infantile par kg de poids corporel représente une contrainte supplémentaire en pédiatrie.

Une nouvelle présentation galénique apparue il y a quelques années permet de satisfaire certaines de ces exigences. Elle consiste en une fine semoule constituée de microsphères dans lesquelles l'ingrédient actif est le plus souvent recouvert d'un film isolant. Cette fine semoule, saupoudrée sur une cuillerée d'aliment semi-solide, par exemple purée, compote, yaourt, est administrée telle quelle.

Toutefois, la forme pharmaceutique dite microsphère se prête difficilement à une libération prolongée de l'ingrédient actif. En effet, à masse égale, la surface développée de ces sphères sera d'autant plus importante que leur diamètre sera plus faible, ce qui aura pour conséquence la dissolution d'autant plus rapide de ces microsphères.

Pour pallier cet inconvénient, il sera généralement recommandé de pourvoir ces sphères d'un enrobage approprié permettant la libération retardée souhaitée.

Si l'enrobage de microsphères pharmaceutiques est industriellement réalisable, il est cependant long car il nécessite une quantité importante de polymère à déposer.

Divers modes de fabrication de ces microsphères, parfois nommées "prills", sont connus et ont été expérimentés pour divers ingrédients actifs.

L'un d'entre eux, appliqué à l'acide valproïque, a été décrit dans « Drug Development and Industrial Pharmacy », 21 (7), pp 793-807 (1995).

Selon ce procédé, on agite un mélange constitué de cire blanche d'abeille sous forme fondue, d'acide valproïque et d'un agent tensio-actif dans un milieu aqueux à pH=4,5 tout en maintenant le mélange à température supérieure à la température de fusion de la cire. Par refroidissement, les particules sphériques formées par dispersion se solidifient en microsphères.

Toutefois, la concentration moyenne de ces microsphères ne dépasse pas 17% en acide valproïque, celles-ci n'étant pas exemptes d'une certaine concentration du tensio-actif utilisé à savoir un mélange de polysorbates éthoxylés ou non éthoxylés.

Une autre technique de formation de microsphères pharmaceutiques fait appel à la technique de stalagmopoièse plus communément dénommée "prilling".

Selon cette technique, décrite notamment dans le brevet DE 2725924, on fait fondre un excipient, dont le point de fusion est inférieur à 120°C, dans lequel on peut ajouter l'ingrédient actif dissous ou dispersé puis on fait passer cette dispersion fondue à travers une buse en vibration provoquant la rupture du jet et la formation de gouttelettes sphériques qui, en tombant, se refroidissent en microsphères.

Ce procédé a été appliqué par exemple à des ingrédients pharmaceutiques de point de cristallisation non défini et fondus dans l'excipient, comme décrit dans le brevet EP 438350. Cet excipient peut être par exemple, un alcool gras tel que l'alcool stéarylique, un acide gras tel que l'acide stéarique, un ester de glycérol, une huile hydrogénée, un sel d'acide gras, un polyol, une cire, un polyoxyéthylèneglycol, un polyoxyéthylène estérifié. L'acide stéarique y est d'ailleurs exemplifié comme excipient.

Toutefois, on y a précisé que dans le cas particulier du kétoprofène seuls pourront être utilisés les acides gras et leurs sels, les esters du glycérol, les huiles hydrogénées, les cires, les polyoxyéthylènes estérifiés.

D'autres ingrédients pharmaceutiques ont été expérimentés également dans la technique de stalagmopoièse.

Ainsi, on a rapporté dans le "Bulletin technique" N° 83, pp 33-47 (1990) de la société Gattefossé, des essais de fabrication de microsphères contenant la théophylline comme ingrédient actif, en utilisant comme excipient matriciel des associations telles qu'acide stéarique / cire blanche, ou cire de camauba / stéarate de glycéryle (PRECIROL ® WL 2155), avec addition ou pas de glycéride polyglycolysé saturé (GELUCIRE® 50-13) en tenant compte des caractéristiques de ces excipients avec le procédé choisi.

Toutefois, des problèmes d'homogénéité ont été enregistrés qui ont conduit, selon cette publication, à abandonner les mélanges dits de cires pour la formation de microsphères à base de théophylline.

Dans le cadre de la présente invention, on a tenté d'appliquer cette technique au valproate sodique. Cependant, les premiers essais effectués à partir d'une matrice comprenant un dérivé stéarique ont mis en évidence un certain nombre de problèmes dont la cristallisation du valproate sodique dans le mélange soumis à la stalagmopoièse et une viscosité inadaptée à la fabrication de microsphères.

Ces inconvénients se sont manifestés notamment lors de l'utilisation d'un mélange 68/2/30 en poids d'alcool stéarylique/produit GELUCIRE® 50-13 / valproate sodique.

D'autre part, des essais mettant en oeuvre le valproate sodique et l'acide stéarique comme excipient ont montré une incompatibilité importante puisque l'on enregistre une précipitation engendrée par le contact de ces ingrédients.

Il apparaît par conséquent illusoire de transposer au valproate sodique les excipients matriciels couramment utilisés dans l'art antérieur pour la préparation de microsphères pharmaceutiques par la technique de stalagmopoièse.

En outre, les essais infructueux rapportés d'une part dans l'état de la technique et d'autre part dans le cadre de la présente invention montrent de manière probante qu'il n'existe pas d'excipient type à pouvoir mettre en oeuvre dans un procédé de stalagmopoièse quelque soit l'ingrédient pharmaceutique actif utilisé.

En conséquence, la mise au point d'une forme d'administration du valproate sodique qui soit à la fois d'usage aisé en pédiatrie et gériatrie, capable de masquer le goût désagréable de cet ingrédient actif et dotée préférentiellement d'un profil de libération prolongé, reste d'un intérêt incontestable.

Or, on a maintenant trouvé de manière surprenante qu'il est possible d'éviter les inconvénients rapportés précédemment en utilisant, comme forme d'administration, des microsphères contenant comme ingrédient actif le valproate sodique ou tout autre sel pharmaceutiquement acceptable de l'acide valproïque et l'acide valproïque lui-même, associé à un support matriciel convenablement choisi.

Dans la plupart des cas, ces microsphères se sont, en outre, révélées adaptées pour une libération prolongée de l'ingrédient actif sans nécessité de les pourvoir d'un enrobage particulier à cet effet.

Ainsi, un premier objet de l'invention conceme des microsphères pharmaceutiques contenant, comme principe actif, un mélange d'acide valproïque et d'un de ses sels pharmaceutiquement acceptables tel que le sel sodique, en association à un support matriciel choisi parmi des esters de glycérol, des huiles hydrogénées, des polyéthylèneglycols estérifiés, des cires et leurs mélanges.

Un autre objet de l'invention conceme un procédé pour la préparation de microsphères contenant un mélange d'acide valproïque et d'un sel pharmaceutiquement acceptable de cet acide, en association à un support matriciel.

Enfin, l'invention se rapporte à une forme pharmaceutique pour administration orale contenant des microsphères selon l'invention.

Dans le présent contexte, aussi bien dans la description que dans les revendications, on désignera par "principe actif" le mélange d'acide valproïque et de sel pharmaceutiquement acceptable de cet acide utilisé dans le cadre de la présente invention.

Les microsphéres de l'invention peuvent être obtenues selon le procédé de stalagmopoiése évoqué précédemment qui consiste :
- à ajouter l'acide valproïque et le sel pharmaceutiquement acceptable de cet acide au support matriciel sous forme fondue, de manière à réaliser une dissolution de ces ingrédients actifs dans la matrice, et maintenir sous agitation le mélange résultant jusqu'à obtention d'un fluide limpide,
- à forcer le mélange sous forme limpide ainsi obtenu à travers une buse subissant une vibration, ce par quoi des gouttelettes sont formées au sortir de la buse et entraînées par gravitation dans une tour dans laquelle circule à contre-courant un gaz froid, généralement de l'air réfrigéré,
- à collecter les microsphères dans le bas de la tour.

Si nécessaire, on peut adjoindre à cette tour de stalagmopoièse un lit fluidisé qui permet de maintenir en fluidisation permanente, les microsphères non encore totalement solidifiées.

Pratiquement, le mélange utilisé dans la stalagmopoièse peut être obtenu en chauffant le support matriciel jusqu' à sa température de fusion puis, lorsque la masse est totalement fondue, en ajoutant l'acide valproïque puis un sel pharmaceutiquement acceptable de cet acide et en maintenant l'ensemble sous agitation jusqu'à obtention d'un fluide limpide.

D'une manière alternative, on peut également préparer ce mélange en ajoutant, de manière séparée, le sel pharmaceutiquement acceptable d'acide valproïque à l'acide valproïque lui-même puis en introduisant l'ensemble dans le support matriciel en fusion.

Les microsphéres de l'invention contiennent, comme principe actif, un mélange, en toutes proportions d'acide valproïque et d'un de ses sels pharmaceutiquement acceptables.

Ce sel est généralement un sel de métal alcalin, de préférence le sel sodique, ou un sel de métal alcalino-terreux tel que le sel de calcium ou de magnésium.

De manière préférentielle mais non exclusive, on utilise en tant que principe actif des mélanges contenant au moins 5% en poids soit d'acide valproïque soit d'un de ses sels pharmaceutiquement acceptables, le surplus étant constitué d'au maximum 95% en poids d'un sel pharmaceutiquement acceptable d'acide valproïque ou d'acide valproïque respectivement.

En particulier le principe actif est constitué de mélanges de 15% à 60% en poids d'acide valproïque et de 40% à 85% en poids de sel pharmaceutiquement acceptable de cet acide.

Des mélanges contenant de 25% à 35% en poids d'acide valproïque et de 65% à 75% en poids de sel en question représentent des mélanges particulièrement préférés aux fins de l'invention.

En règle générale, la viscosité de la composition formée par l'ingrédient pharmaceutique et l'excipient matriciel constitue un facteur limitant pour la mise en oeuvre du procédé de stalagmopoièse adapté à la préparation de microsphères pharmaceutiques.

Dans le cas présent et pour cette raison, les microsphères de l'invention contiendront au maximum 35% en poids de principe actif, tel que décrit précédemment, de préférence de 30% à 35% en poids.

En fait, on a remarqué qu'une concentration pondérale supérieure à 35% en principe actif nécessiterait une température plus élevée de la masse matricielle pour maintenir une viscosité suffisante de celle-ci en vue de préparer les microsphères. D'autre part, une concentration en principe actif supérieure à 35% provoque des difficultés de dissolution de celui-ci dans le support matriciel.

Ce support matriciel, qui est constitué d'un ou de plusieurs excipients de la classe des esters de glycerol, des huiles hydrogénées, des polyéthylèneglycols estérifiés ou des cires, est choisi de telle manière que son point de fusion soit compris entre 50°C et 120°C, généralement entre 70°C et 90°C.

Ce support matriciel est exempt de tout ajout supplémentaire d'agents tensioactifs tels que ceux du genre polysorbates éthoxylés ou non éthoxylés.

De préférence, on sélectionne des excipients susceptibles de fondre à une température de l'ordre de 80°C, celle-ci correspondant à la température qui permet la dissolution du principe actif dans le support matriciel ainsi qu'à la température maximale utilisable pour une bonne conservation des microsphères formées. D'autre part, une température trop élevée nécessiterait une hauteur de chute des microsphères trop importante pour parvenir à la solidification complète tout en risquant de provoquer des dégradations du principe actif.

En tenant compte de cette gamme de température de fusion recommandée, les excipients en question pourront être choisis parmi :
- des glycérides d'acide gras, saturés ou non, en particulier des glycérides contenant jusqu'à 80 atomes de carbone tels que par exemple le tribéhénate de glycéryle, le palmitostéarate de glycéryle, le monostéarate de glycéryle, le monooléate de glycéryle, les glycérides caprylocapriques tels que le tricaprylocaprate de glycéryle.
- des glycerides polyglycolysés saturés tels que des mélanges de monoesters, diesters et triesters de glycérol, et de mono et diesters de polyéthylèneglycol
- des huiles hydrogénées telles que l'huile de ricin hydrogénée
- une cire telle que la cire naturelle d'abeille ou la cire synthétique d'abeille

A titre d'exemple, des excipients matriciels capables de former des microsphères, selon l'invention, peuvent être choisis parmi :
- le tribéhénate de glycéryle tel que commercialisé sous la marque COMPRITOL® 888
- le palmitostéarate de glycéryle tel que commercialisé sous la marque PRECIROL® ATO5
- une huile de ricin hydrogénée telle que commercialisée sous la marque CUTINA® HR
- le monostéarate de glycéryle tel que commercialisé sous la marque MONOMULS® 90/25
- le monooléate de glycéryle tel que commercialisé sous la marque MYVEROL® 18/99
- le tricaprylocaprate de glycéryle tel que commercialisé sous la marque LABRAFAC® lipophile
- des glycérides polyglycolysés saturés tels que commercialisés sous les marques GELUCIRE® 50-02 ou LABRAFIL® 2130CS
- une cire naturelle d'abeille ou une cire synthétique d'abeille telle que commercialisée sous la marque CUTINA® BW
- une paraffine

Les excipients utilisés dans le cadre de l'invention seront sélectionnés, par ailleurs, en tenant compte de la nature hydrophile, lipophile (ou hydrophobe) ou amphiphile qu'ils peuvent posséder et selon la cinétique souhaitée pour la libération du principe actif.

En particulier, et de manière non limitative des mélanges d'excipients du type :
soit tribéhénate de glycéryle (COMPRITOL® 888) / tensio-actif LABRAFIL® 2130CS
soit tribéhénate de glycéryle (COMPRITOL® 888) / monooléate de glycéryle (MYVEROL® 18/99)
soit cire synthétique d'abeille (CUTINA® BW) / huile de ricin hydrogénée (CUTINA® HR) / monostéarate de glycéryle (MONOMULS® 90/25)
se sont révélés adaptés pour un relâchement plutôt immédiat du principe actif.

A titre d'exemple, on a pratiqué des tests de dissolution **in vitro** à pH=6,8 durant 6 heures selon la méthode décrite dans la monographie "Essais de dissolution des formes orales solides" de la Pharmacopée Européenne II dans la partie Prescriptions Générales V. 5.4 pp 1-9 (1995).

A cette fin, on a utilisé des microsphères comprenant de 55% à 60% en poids de produit COMPRITOL® 888 ou de produit PRECIROL® ATO5, de 30% à 35% en poids de principe actif (acide valproïque / valproate sodique) et de 10% à 15% en poids de tensio-actif LABRAFIL® 2130CS

Après 1 heure, on a enregistré 100% de principe actif dissous dans le cas de la formulation incluant le produit PRECIROL® ATO5 et 85% pour celle comprenant le produit COMPRITOL® 888.

De même, des tests de dissolution pratiqués dans les mêmes conditions avec des microsphères contenant de 20% à 25% en poids de produit CUTINA® HR, 20% à 25% en poids de produit CUTINA® BW, 20% à 25% en poids de produit MONOMULS® 90/25 et 30% à 35% en poids de principe actif (acide valproïque / valproate sodique) ont montré une dissolution de 100% du principe actif après 1 heure.

Par ailleurs, un essai analogue effectué avec 55% à 60% en poids de produit COMPRITOL® 888, 30% à 35% en poids de principe actif (acide valproïque / valproate sodique) et 10% en poids de produit MYVEROL® 18/99 a également révélé un profil de libération immédiate.

A la suite de ces essais, on a pu conclure qu'aucune des formulations testées et exemplifiées précédemment, ne présente un profil de libération retardée du principe actif sur une période de 6 heures.

Par contre, d'autres formulations de microsphéres faisant appel au produit COMPRITOL® 888 comme seul excipient matriciel, se sont révélées plutôt adaptées pour une libération retardée du principe actif.

L'addition de certaines substances hydrophobes telles que l'huile de ricin hydrogénée (CUTINA® HR), cire d'abeille, paraffine, produit GELUCIRE® 50-02, produit LABRAFAC® lipophile a permis, par ailleurs, d'accentuer le relâchement différé du principe actif.

A titre d'exemple, des microsphères comportant de 65% à 70% en poids de produit COMPRITOL® 888 et de 30% à 35% en poids de principe actif (acide valproïque / valproate sodique) ont révélé, lors de tests de dissolution un profil de dissolution ralenti. Des résultats analogues ont été enregistrés à partir de microsphères comprenant de 55% à 60% en poids de produit COMPRITOL® 888, 10% à 15% en poids de produit CUTINA® HR, cire d'abeille, paraffine, produit GELUCIRE® 50-02 ou produit LABRAFAC® lipophile et 30% à 35% en poids de principe actif.

En particulier, on a soumis des microsphères de formulations suivantes, à des tests de dissolution à pH=6,8 durant 9 heures selon la méthode de la Pharmacopée Européenne II décrite précédemment, à savoir :

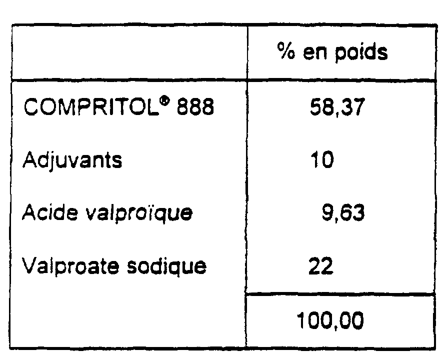

les adjuvants correspondants aux produits CUTINA® HR, CUTINA® BW, paraffine, GELUCIRE® 50-02 et LABRAFAC® lipophile.

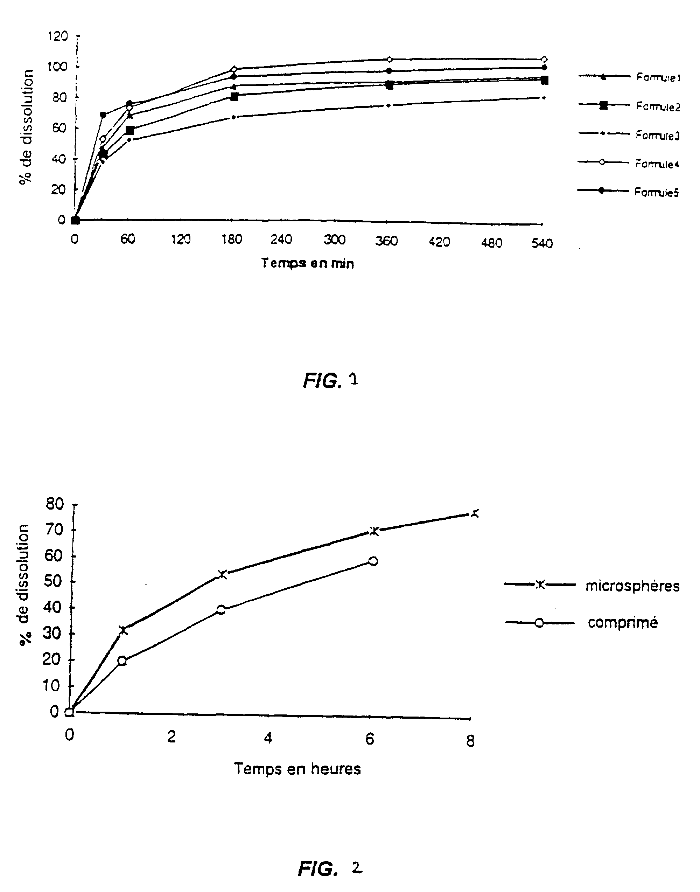

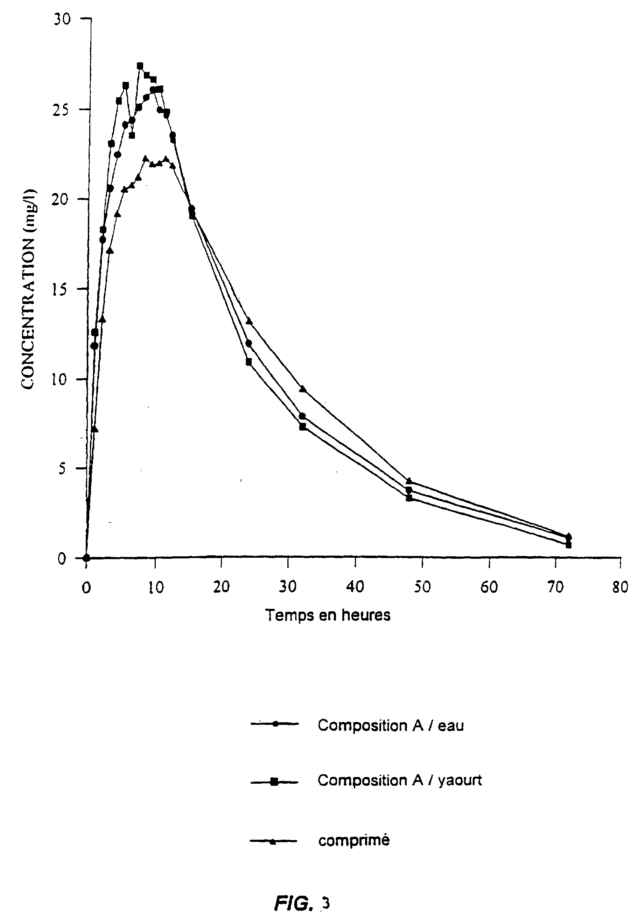

Les profils de dissolution obtenus sont reproduits à la Figure I en annexe sur laquelle:
a) la courbe dénommée "formule 1" représente les pourcentages de dissolution de l'anion valproate en fonction du temps à partir de microsphères ci-dessus dans lesquelles l'adjuvant est le produit CUTINA® HR
b) la courbe dénommée "formule 2" représente des pourcentages analogues à la courbe "formule 1", l'adjuvant étant le produit CUTINA® BW
c) la courbe dénommée "formule 3" représente des pourcentages analogues à la courbe "formule 1", l'adjuvant étant la paraffine
d) la courbe dénommée "formule 4" représente des pourcentages analogues à la courbe "formule 1", l'adjuvant étant le produit GELUCIRE® 50-02
e) la courbe dénommée "formule 5" représente des pourcentages analogues à la courbe "formule 1", l'adjuvant étant le produit LABRAFAC® lipophile.

Les résultats obtenus montrent une cinétique de libération retardée du principe actif. On peut en déduire que les formulations comportant la paraffine, l'huile de ricin hydrogénée (CUTINA® HR) et la cire synthétique d'abeille (CUTINA® BW) apparaissent comme les plus intéressantes.

Le principe actif utilisé dans la présente invention étant constitué d'un composant hydrophobe à savoir l'acide valproïque et d'un composant hydrophile c'est-à-dire un sel pharmaceutiquement acceptable de cet acide, il semble que les excipients matriciels les mieux adaptés devraient être des substances amphiphiles afin d'assurer, par leur partie hydrophobe, la libération retardée du principe actif et par leur partie hydrophile, la régulation du débit de relâchement de ce principe actif à partir de l'excipient.

Or, on a trouvé de manière inattendue qu'un excipient matriciel formé uniquement de cire naturelle d'abeille, corps gras typiquement hydrophobe, permet un profil de relâchement du principe actif parfaitement compatible avec une formulation pharmaceutique retard. Ce profil s'est en outre avéré assez semblable au profil de libération enregistré avec des comprimés connus comportant comme ingrédient actif un mélange d'acide valproïque / valproate sodique.

En particulier, on a effectué des tests de dissolution ainsi que des tests de pharmacocinétique à partir :
- soit de microsphères formées de 68,37 g de cire naturelle d'abeille comme support matriciel et de 31,63 g de principe actif à savoir 9,63 g d'acide valproïque et 22 g de valproate de sodium, ci après dénommées "microsphères de Composition A" Le principe actif correspond en conséquence à un mélange acide valproïque / valproate sodique 30,45% / 69,55% en poids
- soit d'un comprimé sécable retard formulé à 478 mg d'un mélange acide valproïque / valproate sodique à savoir 145 mg d'acide et 333 mg de sel, ce comprimé comprenant notamment des polymères d'acides méthacryliques à titre d'excipient.

Les ingrédients actifs de ce comprimé commercialisé correspondent, en conséquence, à un mélange acide valproïque / valproate sodique 30.33% / 69,67% en poids ou à 500 mg d'ingrédient actif exprimé en valproate sodique.

### I. - Tests de dissolution

Ces tests ont été effectués in vitro à pH= 6,8 selon la méthode citée précédemment

Les profils de dissolution obtenus sont reproduits à la Figure II en annexe sur laquelle :
a) la courbe dénommée "microsphères" représente les pourcentages de dissolution de l'anion valproate en fonction du temps à partir des microsphères de Composition A ci-dessus
b) la courbe dénommée "comprimé" représente les pourcentages de dissolution de l'anion valproate en fonction du temps à partir du comprimé sécable retard ci-dessus.

Les résultats obtenus montrent clairement que le profil de dissolution des microsphères de Composition A est proche et d'allure semblable au profil de dissolution du comprimé puisqu'un écart de 10% seulement les séparé dès la première heure, écart qui reste constant durant au moins 5 heures.

### II. - Tests de pharmacocinétique

Des tests ont été pratiqués sur trois séries de 24 sujets sains auxquels on a administré :
- des microsphères de Composition A dans de l'eau
- des microsphères de Composition A dans du yaourt
- un comprimé sécable retard tel que ci-dessus
de manière que chaque sujet reçoive une dose d'ingrédient actif (acide valproïque/valproate sodique) équivalent à 500 mg exprimés en valproate sodique.

Chez chaque sujet, on a alors enregistré toutes les heures, à partir de l'administration, la concentration sanguine en anion valproate et notamment la concentration sanguine maximale (C max), le temps après l'administration auquel survient cette concentration maximale (T max) et la concentration sanguine résiduelle 24 heures après administration.

Les résultats obtenus sont reproduits à la Figure III en annexe sur laquelle :
a) la courbe dénommée "Composition A / eau" représente la concentration sanguine moyenne obtenue avec les microsphères de Composition A dans l'eau
b) la courbe dénommée "Composition A / yaourt" représente la concentration sanguine moyenne obtenue avec les microsphères de Composition A dans la yaourt
c) la courbe dénommée "comprimé" représente la concentration sanguine moyenne obtenue avec le comprimé sécable retard.
Ces résultats montrent que le C max des microsphères de Composition A est légèrement supérieur à celui du comprimé sécable retard soit environ 16% pour les microsphères dans l'eau et environ 22% pour les microsphères dans le yaourt tandis que les T max se révèlent légèrement plus courts pour les microsphères par rapport au comprimé.

Enfin, les résultats des analyses de variance montrent qu'il n'y a pas de différence significative entre les aires sous les courbes (AUC).

En d'autres termes, ces tests permettent de conclure qu'il n'existe pas de différence significative de biodisponibilité entre les microsphéres de Composition A et le comprimé sécable retard.

En conséquence, les microsphères contenant de 30% à 35% de principe actif en association à un support matriciel constitué dans sa totalité de cire d'abeille, représentent des microsphères d'intérêt essentiel.

De même, des microsphères de ce type, dans lesquelles le principe actif est formé de 25% à 35% d'acide valproïque et de 65% à 75% d'un sel pharmaceutiquement acceptable de cet acide tel que le valproate sodique, sont particulièrement préférées aux fins de l'invention.

A l'inverse des autres technologies de production de sphéroïdes, la stalagmopoièse permet d'obtenir une granulométrie dite monodisperse avec pour avantage la production de sphères régulières en taille. Par conséquent, lors de l'administration du médicament les contenant, la quantité de telles microsphères reçues par le patient sera constante.

Les microsphères pharmaceutiques de l'invention sont dotées de cet avantage en ce qu'elles présentent une telle forme sphérique régulière, d'un diamètre compris entre 250 µm et 500 µm, généralement de l'ordre de 400 µm.

Si nécessaire, ces microsphères peuvent être recouvertes d'un agent filmogène de manière à former par exemple un film gastro-résistant.

En outre, elles peuvent être conditionnées sous différentes formes pharmaceutiques, unitaires ou non, convenant à l'administration orale. De telles formes pharmaceutiques peuvent être par exemple un comprimé, sécable ou non, une capsule, une gélule, une poudre conditionnée par exemple en sachet ou en système distributeur de doses unitaires notamment un flacon distributeur / doseur ou un récipient muni d'une cuillère doseuse à volume réglable par exemple pour l'ajustement de doses au poids du patient.

Lors de leur mise en forme pharmaceutique, les microsphères en question peuvent recevoir des agents facilitant l'écoulement, de même que des lubrifiants, des charges minérales telles que silices, talc, oxyde d'aluminium ou encore des édulcorants tel que l'aspartame.

Ces formes pharmaceutiques pourront avantageusement comprendre par unité d'administration de 50 à 500 mg de principe actif sous forme de microsphères selon l'invention, notamment de 50 à 250 mg.

Les Exemples, non limitatifs, suivants illustrent l'invention :

### EXEMPLE 1

### Préparation de microsphères d'acide valproïque / valproate sodique dans la cire d'abeille

On prépare des microsphères de composition suivante :

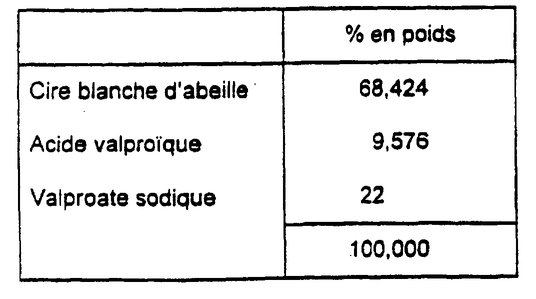

en opérant de la manière suivante.

Dans la cuve double paroi d'un fondoir thermostaté réglé à 95°C, on introduit 68,424g de cire blanche d'abeille puis on fait fondre à la température de 90°C à 93°C tout en contrôlant la fusion totale du produit.

Sous agitation lente, on disperse alors dans la cire fondue maintenue à 90°C, 9,576 g d'acide valproïque puis 22,000 g de valproate de sodium et on vérifie la dissolution des deux ingrédients lorsque le mélange devient limpide (aucune apparition de cristaux).

On procède ensuite à la stalagmopoièse du mélange, à la température de 90°C, à travers des injecteurs de 200 µm maintenus à la température de 87°C à 91°C, et sous 0,5 bar de pression. On règle la fréquence du vibreur de l'appareil (5,75. 10³ à 6,70. 10³Hz), de façon à individualiser les gouttelettes formées, sous contrôle stroboscopique à la fréquence 25000Hz.

On recueille ainsi, à la base de l'appareil, des microsphères solidifiées par refroidissement lors de leur chute dans l'air (hauteur de chute : environ 2,5 m) réfrigéré par un contre courant d'air froid ou par un bain d'azote liquide. Ces microsphères ont un diamètre moyen de 400 µm.

De même manière, mais en utilisant d'autres excipients matriciel, on a préparé des microsphères à partir des formulations suivantes qui se sont toutes révélées limpides après la dissolution du principe actif (aucune apparition de cristaux) :

### EXEMPLE 2

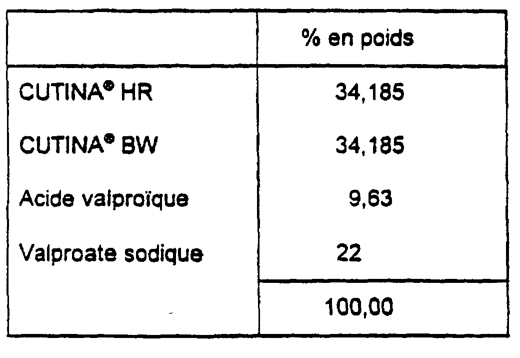

### EXEMPLE 3

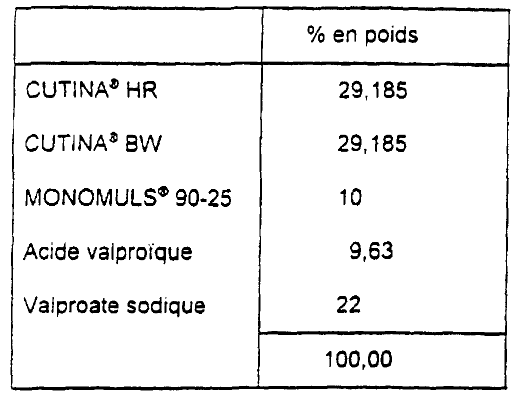

### EXEMPLE 4

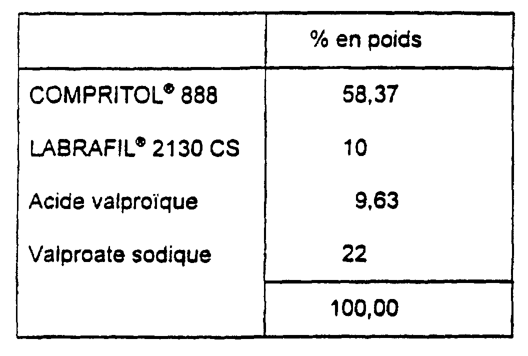

### EXEMPLE 5

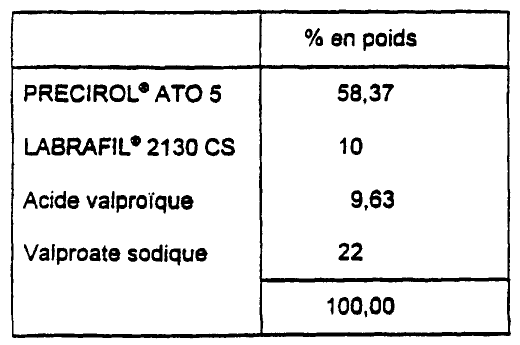

### EXEMPLE 6

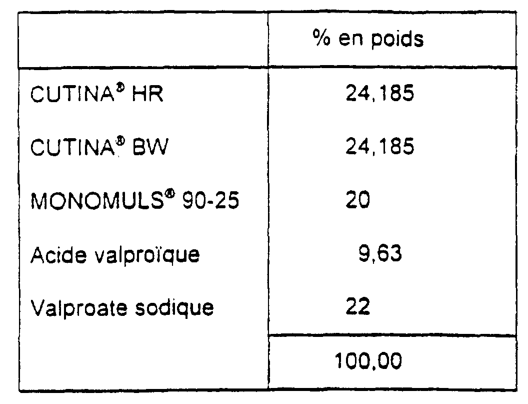

### EXEMPLE 7

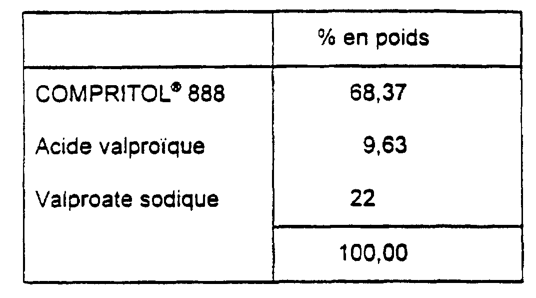

### EXEMPLE 8

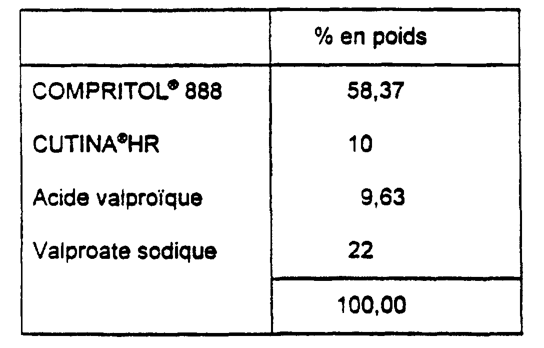

### EXEMPLE 9

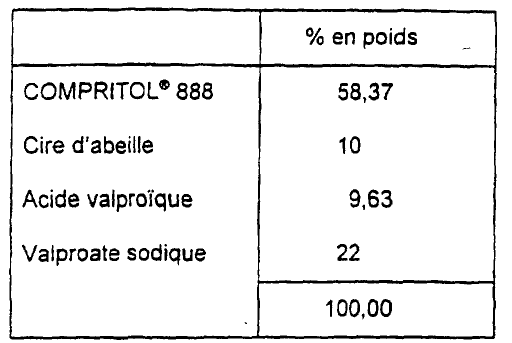

### EXEMPLE 10

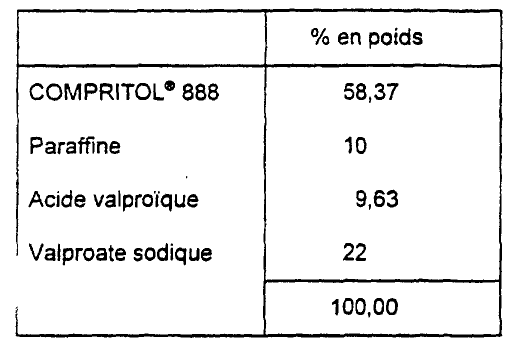

### EXEMPLE 11

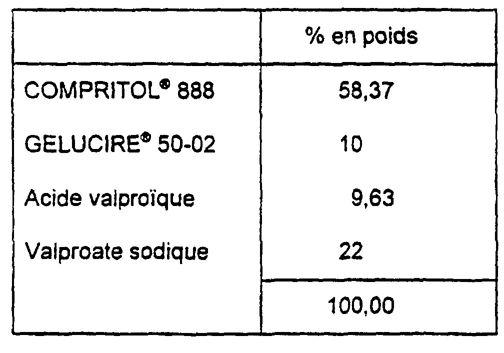

### EXEMPLE 12

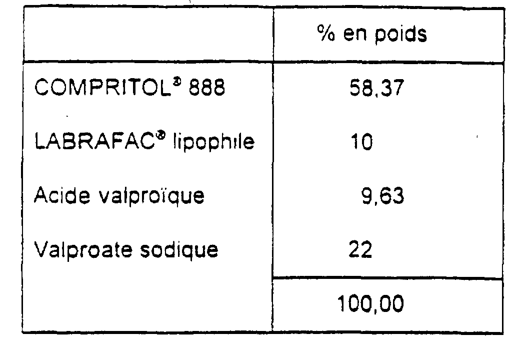

### EXEMPLE 13

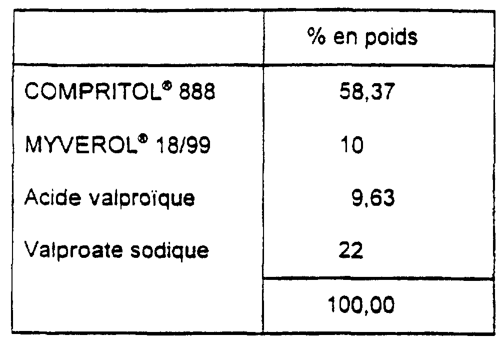

### EXEMPLE 14

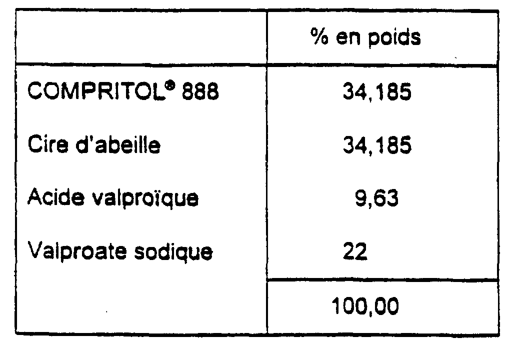

### EXEMPLE 15

### Formes pharmaceutiques contenant des microsphères

### 1) Sachet

On introduit dans un sachet, 756,7 mg de microsphères telles que préparées à l'Exemple 1 de manière à former une unité d'administration orale contenant 238,96 mg de principe actif constitué d'acide valproïque / valproate de sodium 30,4% / 69,6% en poids, ce qui correspond à 250 mg de principe actif exprimé en valproate de sodium.

### 2) Flacon distributeur / doseur

Dans un flacon d'un volume utile d'environ 45 ml, permettant la distribution de doses unitaires, on introduit 24 g de microsphères préparées à l'Exemple 1 soit 8 g de principe actif par unité conditionnée.
Ce flacon permet de distribuer environ 50 doses unitaires contenant 150 mg de principe actif constitué d'acide valproïque / valproate de sodium 30,4% / 69,6%, en poids.

## Revendications

1. Microsphères pharmaceutiques, **caractérisées en ce qu'**elles contiennent, comme principe actif, un mélange d'acide valproïque et d'un de ses sels pharmaceutiquement acceptables, en association à un support matriciel choisi parmi des esters de glycérol, des huiles hydrogénées, des polyéthylèneglycols estérifiés ou des cires et leurs mélanges.

2. Microsphères pharmaceutiques selon la Revendication 1, **caractérisées en ce que** le sel pharmaceutiquement acceptable est un sel de métal alcalin ou un sel de métal alcalino-terreux.

3. Microsphères pharmaceutiques selon la Revendication 1 ou 2, **caractérisées en ce que** le sel de métal alcalin est le sel de sodium.

4. Microsphères pharmaceutiques selon la Revendication 1 ou 2, **caractérisées en ce que** le sel de métal alcalino-terreux, est le sel de calcium ou de magnésium.

5. Microsphères pharmaceutiques selon une des Revendications 1 à 4, **caractérisées en ce que** le principe actif est constitué d'au moins 5% en poids soit d'acide valproïque soit de sel pharmaceutiquement acceptable de cet acide.

6. Microsphères selon une des Revendications 1 à 5, **caractérisées en ce que** le principe actif est constitué d'un mélange de 15% à 60% en poids d'acide valproïque et de 40% à 85% en poids de sel pharmaceutiquement acceptable de cet acide.

7. Microsphères selon une des Revendications 1 à 6, **caractérisées en ce que** le principe actif est constitué d'un mélange de 25% à 35% en poids d'acide valproïque et de 65% à 75% en poids de sel pharmaceutiquement acceptable de cet acide.

8. Microsphères selon une des Revendications 1 à 7, **caractérisées en ce qu'**elles contiennent au maximum 35% en poids de principe actif.

9. Microsphères selon une des Revendications 1 à 8, **caractérisées en ce qu'**elles contiennent de 30% à 35% en poids de principe actif.

10. Microsphères pharmaceutiques selon une des Revendications 1 à 9, **caractérisées en ce que** :
- les esters de glycérol sont des glycérides d'acide gras, saturés ou non, contenant jusqu'à 80 atomes de carbone
- les polyéthylèneglycols estérifiés sont des glycérides polyglycolysés saturés.

11. Microsphères pharmaceutiques selon la Revendication 10, **caractérisées en ce que** les glycérides polyglycolysés saturés sont des mélanges de monoesters, diesters et triesters de glycérol, et de mono et diesters de polyéthylèneglycol.

12. Microsphères pharmaceutiques selon une des Revendications 1 à 11, **caractérisées en ce que** :
- l'ester de glycérol est le tribéhénate de glycéryle, le palmitostéarate de glycéryle, le monostéarate de glycéryle, le monooléate de glycéryle, des glycérides caprylocapriques,
- l'huile hydrogénée est l'huile de ricin hydrogénée
- la cire est la cire naturelle d'abeille ou la cire synthétique d'abeille ou une paraffine.

13. Microsphères pharmaceutiques selon une des Revendications 1 à 12, **caractérisées en ce que** le support matriciel est la cire naturelle d'abeille.

14. Microsphéres pharmaceutiques selon une des Revendications 1 à 13, **caractérisées en ce que** le point de fusion du support matriciel est compris entre 50°C et 120°C.

15. Microsphères pharmaceutiques selon la Revendication 14, **caractérisées en ce que** le point de fusion du support matriciel est compris entre 70°C et 90°C.

16. Microsphères pharmaceutiques selon une des Revendications 1 à 15, **caractérisées en ce qu'**elles contiennent de 30% à 35% de principe actif en association à un support matriciel constitué de cire d'abeille.

17. Microsphères pharmaceutiques selon la Revendication 16, **caractérisées en ce que** le principe actif est formé de 25% à 35% d'acide valproïque et de 65% à 75% d'un sel pharmaceutiquement acceptable d'acide valproïque.

18. Microsphères pharmaceutiques selon la Revendication 16 ou 17, **caractérisées en ce que** le sel pharmaceutiquement acceptable est le sel de sodium.

19. Procédé pour la préparation de microsphères pharmaceutiques selon une des Revendications 1 à 18, **caractérisé en ce que** l'on :
- ajoute l'acide valproïque et le sel pharmaceutiquement acceptable de cet acide au support matriciel sous forme fondue et maintient sous agitation le mélange résultant jusqu'à obtention d'un fluide limpide,
- force le mélange sous forme limpide ainsi obtenu, à travers une buse subissant une vibration, ce par quoi des gouttelettes sont formées au sortir de la buse et entrainées par gravitation dans une tour dans laquelle circule à contre-courant un gaz froid,
- collecte les microsphères dans le bas de la tour.

20. Formes pharmaceutiques pour administration orale, **caractérisées en ce qu'**elles contiennent des microsphères selon une des Revendications 1 à 18.

21. Formes pharmaceutiques selon la Revendication 20, **caractérisées en ce qu'**elles correspondent à des comprimé, capsule, gélule ou poudre conditionnée en sachet ou en système distributeur de doses unitaires.

22. Formes pharmaceutiques selon la Revendication 20 ou 21, **caractérisées en ce qu'**elles contiennent des agents facilitant l'écoulement, des lubrifiants, des charges minérales et/ou des édulcorants.

## Claims

1. Pharmaceutical microspheres, **characterised in that** they contain, as active principle, a mixture of valproic acid and one of the pharmaceutically acceptable salts thereof, combined with a matrix carrier selected from among the glycerol esters, hydrogenated oils, esterified polyethyleneglycole or waxes and mixtures thereof.

2. Pharmaceutical microspheres according to claim 1, **characterised in that** the pharmaceutically acceptable salt is an alkali metal salt or an alkaline earth metal salt.

3. Pharmaceutical microspheres according to claim 1 or 2, **characterised in that** the alkali metal salt is sodium salt.

4. Pharmaceutical microspheres according to claim 1 or 2, **characterised in that** the alkaline earth metal salt is calcium or magnesium salt.

5. Pharmaceutical microspheres according to one of claims 1 to 4, **characterised in that** the active principle consists of at least 5% by weight of either valproic acid or of a pharmaceutically acceptable salt of this acid.

6. Microspheres according to one of claims 1 to 5, **characterised in that** the active principle consists of a mixture of 15% to 60% by weight of valproic acid and 40% to 85% by weight of a pharmaceutically acceptable salt of this acid.

7. Microspheres according to one of claims 1 to 6, **characterised in that** the active principle consists of a mixture of 25% to 35% by weight of valproic acid and 65% to 75% by weight of a pharmaceutically acceptable salt of this acid.

8. Microspheres according to one of claims 1 to 7, **characterised in that** they contain at most 35% by weight of active principle.

9. Microspheres according to one of claims 1 to 8, **characterised in that** they contain 30% to 35% by weight of active principle.

10. Pharmaceutical microspheres according to one of claims 1 to 9, **characterised in that**:
- the glycerol esters are saturated or unsaturated fatty acid glycerides containing up to 80 carbon atoms
- the esterified polyethyleneglycols are saturated polyglycolysed glycerides.

11. Pharmaceutical microspheres according to claim 10, **characterised in that** the saturated polyglycolysed glycerides are mixtures of glycerol monoesters, diesters and triesters and polyethyleneglycol mono- and diesters.

12. Pharmaceutical microspheres according to one of claims 1 to 11, **characterised in that**:
- the glycerol ester is glyceryl tribehenate, glyceryl palmitostearate, glyceryl monostearate, glyceryl monooleate, caprylocapric glycerides,
- the hydrogenated oil is hydrogenated castor oil
- the wax is natural beeswax or synthetic beeswax or a paraffin.

13. Pharmaceutical microspheres according to one of claims 1 to 12, **characterised in that** the matrix carrier is natural beeswax.

14. Pharmaceutical microspheres according to one of claims 1 to 13, **characterised in that** the melting point of the matrix carrier is between 50°C and 120°C.

15. Pharmaceutical microspheres according to claim 14, **characterised in that** the melting point of the matrix carrier is between 70°C and 90°C.

16. Pharmaceutical microspheres according to one of claims 1 to 15, **characterised in that** they contain 30% to 35% of active principle combined with a matrix carrier consisting of natural beeswax.

17. Pharmaceutical microspheres according to claim 16, **characterised in that** the active principle is formed by 25% to 35% of valproic acid and 65% to 75% of a pharmaceutically acceptable salt of valproic acid.

18. Pharmaceutical microspheres according to claim 16 or 17, **characterised in that** the pharmaceutically acceptable salt is the sodium salt.

19. Process for preparing pharmaceutical microspheres according to one of claims 1 to 18, **characterised in that**:
- the valproic acid and the pharmaceutically acceptable salt of this acid are added to the matrix carrier in molten form and the resulting mixture is stirred until a clear fluid is obtained,
- the clear mixture thus obtained is forced through a nozzle subjected to a vibration, so that droplets are formed on leaving the nozzle and carried by gravity into a tower in which a cold gas circulates in countercurrent,
- the microspheres are collected in the base of the tower.

20. Pharmaceutical forms for oral administration, **characterised in that** they contain microspheres according to one of claims 1 to 18.

21. Pharmaceutical forms according to claim 20, **characterised in that** they correspond to tablets, capsules, gelatin capsules or powders packaged in sachets or in a system for delivering single doses.

22. Pharmaceutical forms according to claim 20 or 21, **characterised in that** they contain flow promoters, lubricants, mineral bulking agents and/or sweeteners.

## Patentansprüche

1. Pharmazeutische Mikrokugeln, **dadurch gekennzeichnet, daß** sie als Wirkstoff eine Mischung aus Valprolnsäure und eines ihrer pharmazeutisch annehmbaren Salze in Kombination mit einem Matrixträgermaterial ausgewählt aus Glycerolestern, hydrierten Ölen, veresterten Polyethylenglykolen oder Wachsen und Mischungen davon enthaiten.

2. Pharmazeutische Mikrokugeln nach Anspruch 1, **dadurch gekennzeichnet. daß** das pharmazeutisch annehmbare Salz ein Alkalimetallsalz oder ein Erdalkalimetallsalz ist.

3. Pharmazeutische Mikrokugeln nach Anspruch 1 oder 2, **dadurch gekennzeichnet, daß** das Alkalimetallsalz das Natriumsalz ist.

4. Pharmazentische Mikrokugeln nach Anspruch 1 oder 2. **dadurch gekennzeichnet, daß** das Erdalkalimetallsalz das Calciumsalz oder Magnesiumsalz ist.

5. Pharmazeutische Mikrokugeln nach einem der Ansprüche 1 bis 4, **dadurch gekennzeichnet, daß** der Wirkstoff aus mindestens 5 Gew.-% entweder der Valproinsäure oder des pharmazeutisch annehmbaren Salzes dieser Säure gebildet ist.

6. Mikrokugeln nach einem der Ansprüche 1 bis 5, **dadurch gekennzeichnet, daß** der Wirkstoff aus einer Mischung aus 15 Gew.-% bis 60 Gew.-% Valproinsäure und 40 Gew.-% bis 85 Gew.-% des pharmazeutisch annehmbaren Salzes dieser Säure gebildet ist.

7. Mikrokugeln nach einem der Ansprüche 1 bis 6, **dadurch gekennzeichnet, daß** der Wirkstoff aus einer Mischung aus 25 Gew.-% bis 35 Gew.-% Valproinsäure und 65 Gew.-% bis 75 Gew.-% des pharmazeutisch annehmbaren Salzes dieser Säure gebildet ist.

8. Mikrokugeln nach einem der Ansprüche 1 bis 7, **dadurch gekennzeichnet, daß** sie maximal 35 Gew.-% des Wirkstoffes enthalten.

9. Mikrokugeln nach einem der Ansprüche 1 bis 8, **dadurch gekennzeichnet, daß** sie 30 Gew.-% bis 36 Gew.-% des Wirkstoffes enthalten.

10. Pharmazeutische Mikrokugeln nach einem der Ansprüche 1 bis 9, **dadurch gekennzeichnet.**
- **daß** die Glycerolester Glyceride von gesättigten oder ungesättigten Fettsäuren sind, die bis zu 80 Kohlenstoffatome enthalten and
- die veresterten Polyethylenglycole gesättigte polyglykolisierte Glyceride sind.

11. Pharmazeutische Mikrokugeln nach Anspruch 10, **dadurch gekennzeichnet, daß** die gesättigten polyglykolisierten Glyceride Mischungen sind aus Monoestern, Diestern und Triestern von Glycerol und Mono- und Diestern von Polyethylenglykol.

12. Pharmazeutische Mikrokugeln nach einem der Ansprüche 1 bis 11, **dadurch gekennzeichnet, daß**
- der Glycerolester Glyceryltribehenat, Glycerylpalmitostearat, Glycerylmonostearat, Glycerylmonooleat oder ein Caprylocaprinsäureglycerid ist,
- das hydrierte Öl hydriertes Rizinusöl ist und
- das Wachs natürliches Bienenwachs oder synthetisches Bienenwachs oder ein Paraffin ist.

13. Pharmazeutische Mikrokogeln nach einem der Ansprüche 1 bis 12. **dadurch gekennzeichnet, daß** das Matrixträgermaterial natürliches Bienenwachs ist.

14. Pharmazeutische Mikrokugeln nach einem der Ansprüche 1 bis 13, **dadurch gekennzeichnet, daß** der Schmelzpunkt des Matrixträgermaterials zwischen 50°C und 120°C liegt.

15. Pharmazeutische Mikrokugeln nach Anspruch 14, **dadurch gekennzeichnet, daß** der Schmelzpunkt des Matrixträgermaterials zwischen 70°C und 90°C liegt.

16. Pharmazeutische Mikrokugeln nach einem der Ansprüche 1 bis 15, **dadurch gekennzeichnet, daß** sie 30 % bis 35 % des Wirkstoffs in Kombination mit einem aus Bienenwachs gebildeten Matrixträgermaterial enthalten.

17. Pharmazeutische Mikrokugeln nach Anspruch 16, **dadurch gekennzeichnet, daß** der Wirkstoff aus 25 % bis 35 % Valproinsäure und 65 % bis 75 % eines pharmazeutisch annehmbaren Salzes der Valproinsäure gebildet ist.

18. Pharmazeutische Mikrokugeln nach Anspruch 16 oder 17, dadurch gekennezeichnet, daß das pharmazeutisch annehmbare Salz Natriumsalz ist.

19. Verfahren zur Herstellung der pharmazeutischen Mikrokugeln nach einem der Ansprüche 1 bis 18, **dadurch gekennzeichnet, daß** man:
- Valproinsäure und pharmazeutisch annehmbare Salz dieser Säure zu dem Matrixträgermaterial in geschmolzener Form gibt und die gebildete Mischung rührt bis zum Erhalt einer klaren Flüssigkeit,
- die in der dieser Weise in klarer erhaltene Mischung durch eine der Vibration unterworfene Düse presst, so daß sich am Ausgang der Düse Tröpfchen bilden, welche unter Einwirkung der Schwerkraft in einen Turm eingeführt werden, in dem ein kaltes Gas im Gegenstrom zirkuliert, und
- die Mikrokugeln am Boden des Turmes sammelt.

20. Pharmazeutische Zubereitungen für die orale Verabreichung, **dadurch gekennzeichnet, daß** sie Mikrokugeln nach einem der Ansprüche 1 bis 18 enthalten.

21. Pharmazeutische Zubereitungen nach Anspruch 20, **dadurch gekennzeichnet, daß** sie in Form von Tabletten, Kapseln, Gelkapseln oder eines in einem Sachet vorliegenden Pulver oder in einem System zur Verabreichung von Einheitsdosierungen vorliegen.

22. Pharmazeutische Zubereitungen nach Anspruch 20 und 21, **dadurch gekennzeichnet, daß** sie die Fließeigenschaften verbessernde Bestandteile. Gleitmittel, anorganische Füllstoffe und/oder Süßungsmittel enthalten.
